# EUROPEAN PATENT APPLICATION

(11) **EP 3 550 303 A1**
(43) Date of publication of application: **09.10.2019**
(21) Application number: 17876818.0
(22) Date of filing: 29.11.2017
(51) Int. Cl.: G01N 33/574, G01N 33/48, G01N 33/53

(54) **METHOD FOR ACQUIRING MEDICAL CARE AUXILIARY INFORMATION**

(30) Priority: 29.11.2016 JP 2016231323
(71) Applicant: Konica Minolta, Inc., Tokyo 100-7015 (JP); Hirosaki University, Hirosaki-shi, Aomori 036-8560 (JP)
(72) Inventor: KANEKO Tomonori, Tokyo 100-7015 (JP); KAYA Takatoshi, Tokyo 100-7015 (JP); OHYAMA Chikara, Hirosaki-shi Aomori 036-8560 (JP); YONEYAMA Tohru, Hirosaki-shi Aomori 036-8560 (JP); TOBISAWA Yuki, Hirosaki-shi Aomori 036-8560 (JP)
(74) Representative: MFG Patentanwälte Meyer-Wildhagen Meggle-Freund Gerhard PartG mbB
(86) International application number: PCT/JP2017/042803
(87) International publication number: WO 2018/101328

(57) **Abstract**

The present invention relates to a method for acquiring auxiliary information for diagnosis and treatment (medical care) of prostate cancer. A method for acquiring medical care auxiliary information for estimating a risk of prostate cancer recurrence with a specimen of prostate tissue, wherein an at least three-stage evaluation score is used to express a quantity of biological material in a tumor site of the specimen, the biological material having a β-N-acetylgalactosamine residue at a nonreducing terminal of a sugar chain.

## Description

### Technical Field

The present invention relates to a method for acquiring auxiliary information for diagnosis and treatment (medical care) of prostate cancer.

### Background Art

Prostate cancer, which mainly occurs in men aged 60 years and over, is the second leading cause, after lung cancer, of cancer mortality in European and North American men. Even after treatment intended to cure radically (treatment intended for radical prostatectomy), within 10 years, 35% of patients are found to have elevated blood levels of a prostate-specific antigen (PSA) or a prostate cancer marker. In other words, 35% of patients suffer a recurrence of prostate cancer. Accordingly, to select better treatment for individual prostate cancer cases, there is an idea to prognose and predict each case with accuracy (predict a risk of recurrence), to grade the risk before treatment, and to treat according to the grading.

However, the related art has not established, thus far, such a technique of predicting or estimating a risk of prostate cancer recurrence. For that reason, for example, the status quo requires periodical measurement (follow-up) of a blood level of PSA even after surgery and requires the salvage radiation therapy in case of an abnormality. Alternatively, the adjuvant radiation therapy has been studied as an adjuvant therapy for the prevention of recurrence.

With regard to prostate cancer, there is known a method for staining prostate tissue utilizing a reaction between a specific sugar chain within the prostate tissue of a patient and a lectin having an affinity for the sugar chain (lectin histochemical staining method). For example, as a lectin histochemical staining method using Wisteria floribunda Agglutinin (WFA) or a lectin having an affinity for a β-N-acetylgalactosamine residue, Non-Patent Literature 1 discloses the following technique. That is, prostate tissue is first allowed to react with biotin-labeled WFA and next with a complex of avidin and a biotin-labeled enzyme (a complex obtained by preliminarily reacting avidin with a biotin-labeled enzyme (peroxidase)). The prostate tissue is then allowed to react with diaminobenzidine (DAB) or a substrate corresponding to the enzyme so as to be colored. In this literature, the lectin histochemical staining method is used to stain sections of benign prostate cancer tissue and malignant prostate cancer determined by the Gleason score. This literature aims to study whether the lectin histochemical staining method enables differentiation between malignancy and benignancy of prostate cancer. As a result of the staining, malignant prostate cancer tissue shows good stainability (stainability is estimated in two patterns, positive (+) and negative (-)) and good sensitivity, but 56% of benign prostate cancer tissue also shows positive stainability. Accordingly, the specificity of the staining method is found to be poor, and the effectiveness of the staining method for diagnosis is considered to be low.

However, Non-Patent Literature 1 includes no description of a correlation between a stained image obtained by the lectin histochemical staining method and the risk of prostate cancer recurrence.

### Citation List

### Non Patent Literature

Non Patent Literature 1: McMahon et al., J Clin Pathol, 1992, 45, 1094-1098

### Summary of Invention

### Technical Problem

As described above, the related art has not established, thus far, a method for estimating a risk of prostate cancer recurrence. Acquiring medical care auxiliary information for such an estimation enables the selection of better treatment to prevent prostate cancer recurrence and offers a great benefit to patients.

An object of the present invention is to provide a method for estimating a risk of prostate cancer recurrence.

### Solution to Problem

The inventors of the present invention have expressed a quantity of biological material, which has an affinity for a specific lectin such as WFA, in a tumor site of a prostate tissue specimen collected from a prostate cancer patient, that is, a quantity of biological material having a β-N-acetylgalactosamine residue at a nonreducing terminal of a sugar chain, with a three-stage evaluation score based on a stained image of a tissue section. Furthermore, the inventors have made the evaluation score associated with information indicating whether prostate cancer occurs or does not occur within a predetermined period after radical prostatectomy. Accordingly, the inventors of the present invention have found, with a statistically significant difference, that the evaluation score 1 (evaluation for a specimen with the lowest quantity of biological material among the three stages) indicates a low recurrence risk, and that, conversely, the evaluation score 3 (evaluation for a specimen with the highest quantity of biological material among the three stages) indicates a high recurrence risk. This fact proves that setting an appropriate threshold makes it possible to estimate the recurrence risk of a prostate cancer patient from whom a specimen is taken.

According to an aspect of the present invention, there is provided "a method for acquiring medical care auxiliary information for estimating a risk of prostate cancer recurrence with a prostate tissue specimen, wherein an at least three-stage evaluation score is used to express a quantity of biological material in a tumor site of the specimen, the biological material having a β-N-acetylgalactosamine residue at a nonreducing terminal of a sugar chain."

In other words, according to another aspect of the present invention, there is provided "a method for measuring or evaluating a quantity of biological material in a tumor site of a prostate tissue specimen, the biological material having a β-N-acetylgalactosamine residue at a nonreducing terminal of a sugar chain, wherein the method involves at least binding to the biological material a molecule having an affinity for a β-N-acetylgalactosamine residue so as to stain the biological material; and measuring staining intensity of a specimen prepared by the step of binding and staining." Similar to the above method, the "at least three-stage evaluation score is used to express" results of this measurement method or estimation method. The evaluation score is used, as diagnostic auxiliary information, for the estimation (medical care) of the risk of prostate cancer recurrence.

The method for acquiring medical care auxiliary information according to an embodiment of the present invention is different from a method in the related art as one disclosed in Non-Patent Literature 1 in that a quantity of predetermined biological material is estimated with the "at least three-stage evaluation score." Furthermore, the method according to an embodiment of the present invention is different from the related art in that the obtained medical care auxiliary information is used as the evaluation score associated with the risk of prostate cancer recurrence, which has not been achieved by the related art. Accordingly, it is possible to clearly distinguish the method from one in the related art.

### Advantageous Effects of Invention

With medical care auxiliary information obtained by a method for acquiring medical care auxiliary information according to an embodiment of the present invention, it is possible to estimate a risk of prostate cancer recurrence, which has not been achieved by the related art. The estimation of the recurrence risk enables individual treatment, taking patients' QOL into consideration. For example, it is possible to select adjuvant therapy according to the recurrence risk of each patient.

### Brief Description of Drawings

Fig. 1 is an estimated structural formula of a sugar chain in a PSA contained in a prostate cancer cell. Reference: Fukushima et al. Glycobiology, 20, 4, 452-460 (2010).
Fig. 2 illustrates examples of a DAB-stained image with a WFL status + (right) and an HE-stained image of a specimen slide (left) corresponding to the DAB-stained image.
Fig. 3 illustrates examples of a DAB-stained image with a WFL status ++ (right) and an HE-stained image of a specimen slide (left) corresponding to the DAB-stained image.
Fig. 4 illustrates examples of a DAB-stained image with a WFL status +++ (right) and an HE-stained image of a specimen slide (left) corresponding to the DAB-stained image.
Fig. 5 is a graph illustrating a relationship between the nonrecurrence rate of prostate cancer and the WFL status, prepared in Example according to the Kapran-Meier method.
Fig. 6 is a graph illustrating a relationship between the WFL status and GS studied in Example.
Fig. 7 is a graph illustrating a relationship between the WFL status and pT studied in Example.
Fig. 8 is a graph illustrating a relationship between the WFL status and pn studied in Example.
Fig. 9 is an example of a nomogram prepared in Examples and relating to the prediction of prostate cancer recurrence.
Fig. 10 is an example of a nomogram prepared in Examples and relating to the prediction of prostate cancer recurrence, which is different from one illustrated in Fig. 9.

### Description of Embodiments

According to an embodiment of the present invention, in a method for acquiring medical care auxiliary information for estimating a risk of prostate cancer recurrence, an at least three-stage evaluation score is used to express a quantity of biological material in a tumor site of a prostate tissue specimen, in which the biological material has a β-N-acetylgalactosamine residue at a nonreducing terminal of a sugar chain.

The evaluation score as medical care auxiliary information enables, for example, a doctor to estimate the recurrence risk. "To estimate the recurrence risk" is useful for preventing prostate cancer recurrence and for diagnosis including the selection of better treatment.

The method according to an embodiment of the present invention offers, for example, a doctor with diagnostic auxiliary information when a person other than the doctor (and a person who has received an instruction from the doctor) estimates a quantity of biological material having a β-N-acetylgalactosamine residue at a nonreducing terminal of a sugar chain with the at least three-stage evaluation score.

"Prostate tissue" is a specimen collected from a living body. Prostate tissue may be collected at biopsy (before radical prostatectomy) or at radical prostatectomy. The collected specimen may be sliced to prepare a specimen slide after, for example, formalin fixation and paraffin embedding according to a common procedure and may be used for estimating a quantity of predetermined biological material. A "tumor site" in prostate tissue is distinguished from a "non-tumor site" according to a common procedure such as histopathological grading (Gleason grading system).

A subject of the method for acquiring medical care auxiliary information according to an embodiment of the present invention, or a subject from whom a prostate tissue specimen is taken, is typically a human prostate cancer patient. However, the method may also employ mammals other than human beings such as animal models of human prostate cancer. A human prostate cancer patient is those who require or desire estimation of the risk of prostate cancer recurrence, and the patient may be subjected to radical prostatectomy or may not. Examples of mammals other than human beings include animal models such as mice and rats with prostate cancer.

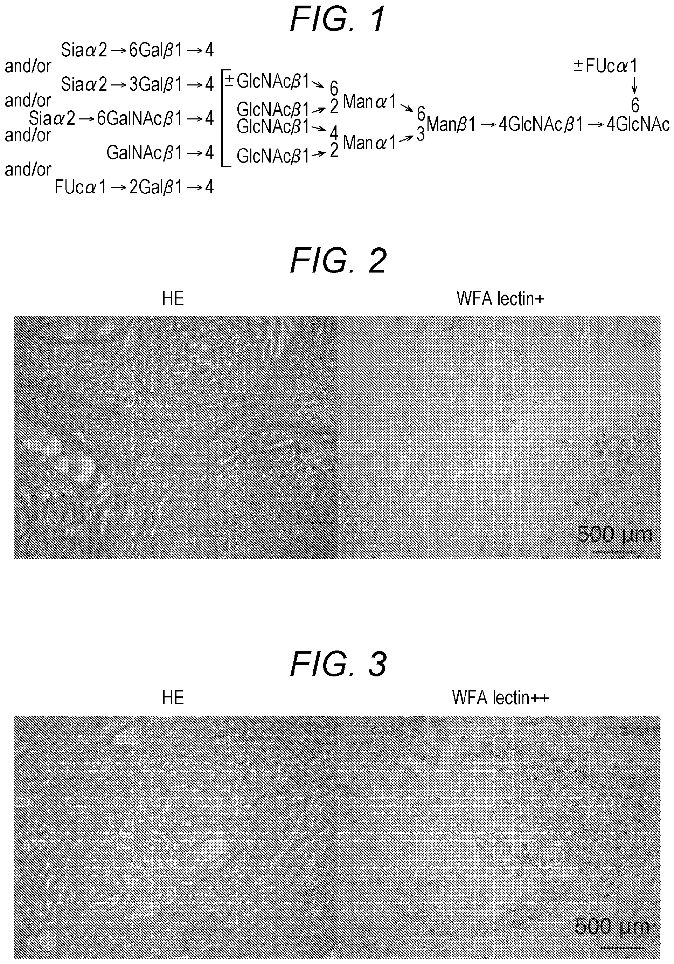

A "biological material having a β-N-acetylgalactosamine residue at a nonreducing terminal of a sugar chain" (which may be referred to herein as "β-GalNAc-biological material")) is a biological material which allows the binding of a molecule having an affinity for a β-N-acetylgalactosamine residue (which may be referred to herein as "molecule having an affinity for a β-GalNAc residue"). The β-GalNAc-biological material is mainly presumed to be a PSA having a β-N-acetylgalactosamine-(1→4)-N-acetylglucosamine residue (a GalNAc(β1→4)GlcNAc residue) at a nonreducing terminal of a sugar chain (which may be referred to herein as "LacdiNAc-PSA") but is not limited thereto. Examples of the β-GalNAc-biological material include glycoprotein, glycolipid, and other biological materials which are included in prostate tissue and which have a β-N-acetylgalactosamine residue at a nonreducing terminal of a sugar chain. Fig. 1 illustrates an estimated structural formula of a sugar chain in a PSA contained in a prostate cancer cell. Such a PSA is presumed to have a sugar chain with a sialylated terminal or a sugar chain with terminal fucose as well as a sugar chain in which a GalNAc residue is bound to a GlcNAc residue by a β1→4 bond at a nonreducing terminal.

A "quantity of β-GalNAc-biological material in a tissue specimen" usually represents a quantity of β-GalNAc-biological material in the entire staining image captured for measuring the quantity and used for analysis. However, as needed, (for example, when the after-mentioned PID method is employed), the quantity also represents a quantity of β-GalNAc-biological material in a specific region of the staining image, for example, a quantity per unit area or a quantity per cell.

For example, in capturing a tissue section stained with a color-producing agent such as DAB by a lectin histochemical staining method, and in quantifying the staining intensity of the color-producing agent with image analysis software, the "at least three-stage evaluation score" may be offered when the staining intensity is within a specific range, referring to a predetermined standard. An example of the image analysis software includes "Image J" (Fiji software, open source) which is quantified by the "reciprocal intensity" (while the staining intensity of an unstained white area is set to "250," the staining intensity of a stained area is expressed by a value of 250 or less. The unit herein is a.u.). A method for estimating the staining intensity is known. For example, see Ngyen et al. (Research Article, 2 (1), 2013).

The evaluation score usually includes a grade group with high recurrence risk and a grade group with low recurrence risk. For example, in expressing with the three-stage evaluation score, two stages among the three stages may represent grade groups with high recurrence risk, and the other one may represent a grade group with low recurrence risk. Alternatively, one stage may be a grade group with high recurrence risk and the other two may be grade groups with low recurrence risk.

Note that the grade group with high recurrence risk is usually a grade group into which the after-mentioned biological material is classified when a quantity of biological material is equal to or larger than a threshold, and the grade group with low recurrence risk is usually a grade group into which the biological material is classified when a quantity of biological material is less than the threshold.

As an embodiment of the present invention, the "at least three-stage evaluation score" is obtained by, as a substitute for the color-producing agent such as DAB, a fluorescent stain including a "fluorescent nanoparticle" that is fluorescently labeled per molecule. The "fluorescent nanoparticle" is, for example, a nanosized phosphor integrated dot (PID) in which phosphors such as fluorochromes or quantum dots are integrated with a matrix such as silica or resin. In this case, the number of bright spots of the fluorescent nanoparticle represents a quantity of biological material having a β-N-acetylgalactosamine residue at a nonreducing terminal of a sugar chain on a tissue section. The "at least three-stage evaluation score" in this embodiment (herein referred to as the "PID method") may be offered specifically when the number of bright spots of the fluorescent nanoparticle is within a specific range. Alternatively, the number of bright spots of the fluorescent nanoparticle may be regarded as a (semi-continuous and three or more-stage) evaluation score. PID is a known fluorescent labeling agent. See, for example, WO 2012/029752 and WO 2013/035703 (refer to these Patent Literatures to see a method for measuring the number of bright spots of PID).

### [Measurement Method]

A method for measuring a quantity of β-GalNAc-biological material, for example, LacdiNAc-PSA, in a prostate tissue specimen is not particularly limited and may employ various measurement methods as long as an accurate measurement value expressed by the "at least three-stage evaluation score" is obtained.

The method for measuring a quantity of β-GalNAc-biological material preferably involves binding of a molecule having an affinity for a β-N-acetylgalactosamine residue (molecule having an affinity for a β-GalNAc residue) to a β-GalNAc-biological material. Furthermore, the molecule having an affinity for a β-GalNAc residue may have a strong affinity for a β-N-acetylgalactosamine residue and may have a weak affinity for substances and structures other than the β-N-acetylgalactosamine residue.

### • Molecule Having Affinity for β-GalNAc Residue

Examples of the molecule having an affinity for a β-GalNAc residue include a lectin having an affinity for a β-GalNAc residue or an antibody that recognizes a β-GalNAc residue as an epitope (anti-β-GalNAc antibody).

### • Lectin Having Affinity for β-GalNAc Residue

A lectin is a protein having an affinity for a specific sugar residue, or a protein that discerns and binds to the specific sugar residue. Many kinds of lectins derived from various organisms (which are also referred to as "agglutinin") are known. There are various kinds of sugar residues having an affinity for different kinds of lectins. Many lectins have an affinity not only for one kind of sugar residue but also for plural kinds (note that the affinity for a specific sugar residue is strong, while the affinity for other sugar residues is weak). A typical antibody such as an anti-β-GalNAc antibody that recognizes a specific sugar residue in a sugar chain as an epitope is difficult to prepare, whereas the lectin having an affinity for a β-GalNAc residue is inexpensive and available in large quantities. Furthermore, due to excellent stability and better keeping qualities, the lectin having an affinity for a β-GalNAc residue is preferable as the molecule having an affinity for a β-GalNAc residue.

Various kinds of lectins having an affinity for a β-GalNAc residue are known, and another lectin may be isolated from a new organism in the future. The present invention may employ any kind of lectin as long as the lectin has a sufficiently strong affinity for a β-GalNAc residue, that is, as long as the lectin has no affinity for other sugar residues or has an affinity for other sugar residues but the affinity is sufficiently weaker than that for a GalNAc residue (for example, the binding constant is lower by several orders), and as long as LacdiNAc-PSA is quantified with a sufficient degree of accuracy.

Specific examples of the lectin having an affinity for a β-GalNAc residue include Wisteria floribunda Agglutinin (WFA), Soybean Agglutinin (SBA), and Vicia Villosa Lectin (VVL). These lectins are separated (extracted) from organisms such as seeds from which they are derived and then purified. Alternatively, commercially available products may be employed.

WFA is a lectin (agglutinin) derived from Wisteria floribunda and is also referred to as Wisteria floribunda Lectin (WFL). WFA has affinities for N-acetyl-D-galactosamine residues (GalNAc), or for both an α-N-acetyl-D-galactosamine residue (a-GalNAc) and a β-N-acetyl-D-galactosamine residue (β-GalNAc). WFA binds to, for example, residues at a nonreducing terminal of a sugar chain such as a GalNAc(α1→6)Gal residue, a GalNAc(α1→3)Gal/GalNAc residue, a GalNAc(β1→4)Gal residue, and a GalNAc(β1→4)GlcNAc residue, and binds to GalNAc-serine or threonine (Ser/Thr) at a reducing terminal of a sugar chain. Note that WFA also has relatively weak affinities for lactose and galactose. As is known, the affinity of WFA for β-GalNAc is strong. Assume that the affinity of WFA for α-GalNAc is 1, the affinity for β-GalNAc is about 10. Furthermore, assume that the affinity of WFA for galactose is 1, the affinity of WFA for β-GalNAc is about 100. (See, for example, J. Biol. Chem. Sep. 2016, M116.750463)

SBA is a lectin (agglutinin) derived from soybean. SBA also has affinities for both an α-N-acetyl-D-galactosamine residue (a-GalNAc) and a β-N-acetyl-D-galactosamine residue (β-GalNAc) (the affinity for the former residue is stronger than that for the latter residue). SBA binds to, for example, residues at a nonreducing terminal of a sugar chain such as a GalNAc(α1→3)Gal residue, a GalNAc(β1→4)Gal residue, and a GalNAc(β1→4)GlcNAc residue. SBA also has a relatively weak affinity for galactose.

VVL is a lectin (agglutinin) derived from Vicia villosa Roth and may also be referred to as Vicia villosa Agglutinin (VVA). VVL also has affinities for both an α-N-acetyl-D-galactosamine residue (a-GalNAc) and a β-N-acetyl-D-galactosamine residue (β-GalNAc). VVL binds to, for example, residues at a nonreducing terminal of a sugar chain such as a GalNAc(α1→3)Gal residue, a GalNAc(β1→4)Gal residue, and a GalNAc(β1→4)GlcNAc residue.

### • Embodiment of Method for Measuring Quantity of β-GalNAc-biological Material

A method using an Avidin Biotinylated Enzyme Complex (ABC) method and the molecule having an affinity for a β-GalNAc residue is an example of a representative embodiment of the method for measuring a quantity of β-GalNAc-biological material which involves binding of the molecule having an affinity for a β-GalNAc residue to the β-GalNAc-biological material. In this method, first, a biotin-labeled molecule having an affinity for a β-GalNAc residue (lectin having an affinity for a β-GalNAc residue or an anti-β-GalNAc antibody) is bound to a β-GalNAc-biological material on a specimen (prostate tissue), and then a complex of avidin and a biotin-labeled enzyme (a complex obtained by preliminarily reacting avidin with a biotin-labeled enzyme) is bound to the biotin-labeled molecule having an affinity for a β-GalNAc residue. The enzyme herein may employ one that is used in a known staining method. Examples of such an enzyme include peroxidase, alkaline phosphatase, and glucose oxidase. Finally, a reaction between the enzyme and a corresponding substrate, for example, a reaction between peroxidase and a corresponding DAB, generates a dye. Accordingly, the periphery of the β-GalNAc-biological material on the specimen is stained. Then, a bright-field image is observed and captured by a microscope at the desired magnification and used for determining the evaluation score. The density of the staining is an index that reflects the abundance of a β-GalNAc-biological material on a specimen.

In an embodiment (preferably, the PID method) using a fluorescent nanoparticle in place of a substrate (color-producing agent) such as DAB, first, a biotin-labeled molecule having an affinity for a β-GalNAc residue is bound to a β-GalNAc-biological material on a prostate tissue specimen, and then an avidin-modified fluorescent nanoparticle (preferably, PID) is bound to the biotin-labeled molecule having an affinity for a β-GalNAc residue. After that, a dark-field fluorescence image is observed and captured by a microscope at the desired magnification and used for determining the evaluation score. The number of bright spots of the fluorescent nanoparticle is an index that reflects the abundance of a β-GalNAc-biological material on a specimen.

The aforementioned measurement method may employ avidin in egg white as avidin, but it is preferable to employ, as a substitute of avidin, streptavidin which has high specificity (little non-specific binding) to biotin or a biotin-binding protein such as neutravidin which has higher specificity (less non-specific binding) to biotin and is obtained by removing a sugar chain from avidin. Alternatively, instead of a reaction between biotin and a biotin-binding protein such as avidin, a reaction between a hapten and an anti-hapten antibody, for example, a hapten such as dinitrophenol and digoxigenin and an antibody against a corresponding hapten may be utilized to label the β-GalNAc-biological material with an enzyme or a fluorescent nanoparticle.

The lectin having an affinity for β-GalNAc labeled with biotin or the like and the fluorescent nanoparticle modified with avidin or the like are prepared by a known method, using a commercially available kit or the like. A substrate (color-producing agent) corresponding to an enzyme and a fluorescent nanoparticle such as PID are not particularly limited, and the present invention may employ ones that emit the desired color.

### [Threshold]

Based on a quantity of β-GalNAc-biological material in the prostate tissue specimen, a threshold (cut-off value) required for acquiring medical care auxiliary information associated with the risk of prostate cancer recurrence is set by a common method similar to a known method for acquiring medical care auxiliary method, similarly, for example, to a threshold used for estimating a predetermined matter with respect to a diagnostic marker or a tumor marker.

For example, aiming at a plurality of patients diagnosed with prostate cancer and subjected to radical prostatectomy, tissue section slides are prepared using prostate tissue of each patients as a specimen, and the tissue section slides are stained by a predetermined method as described above and are subjected to image analysis, whereby acquiring information associated with a quantity of β-GalNAc-biological material. When a quantity of biological material is equal to or more than a threshold, the biological material is classified into a grade group with high recurrence risk of the evaluation score, and when a quantity of biological material is less than the threshold, the biological material is classified into a grade group with low recurrence risk of the evaluation score.

Grade groups are classified into a grade group with high recurrence risk and a grade group with low recurrence risk. For example, in expressing with the three-stage evaluation score, two stages in the three stages may be grade groups with high recurrence risk, and the other one may be a grade group with low recurrence risk. Alternatively, one stage may be a grade group with high recurrence risk and the other two may be grade groups with low recurrence risk.

The evaluation score, for example, granting of the evaluation score may be appropriately adjusted and defined, depending on a numerical value that represents staining density.

The evaluation score is associated with information for each specimen on whether a patient whose prostate cancer has relapsed or not within a predetermined period from the radical prostatectomy. Based on, for example, a relationship between the nonrecurrence rate of prostate cancer by the Kapran-Meier method and the evaluation score, the risk of prostate cancer recurrence is estimated.

In the grade group with high recurrence risk, a quantity of β-GalNAc-biological material is equal to the threshold or more. With a high evaluation score, the nonrecurrence rate of prostate cancer is estimated to be low, that is, the recurrence risk of prostate cancer tends to be high. In the grade group with low recurrence risk, a quantity of β-GalNAc-biological material is less than the threshold. With a low evaluation score, the nonrecurrence rate of prostate cancer is estimated to be high, that is, the recurrence risk of prostate cancer tends to be low.

The following embodiment may be employed as an example. That is, as shown in the following Examples, the evaluation score of the β-GalNAc-biological material is expressed by three stages based on the staining density, and when the evaluation score is 2 or 3, the risk of prostate cancer recurrence is estimated to be high. In determining a criterion of each stage of the evaluation score, it is desirable to determine the stage of the evaluation score after visually comparing the staining density of samples and affirming a clear difference in staining density between the stages of the evaluation score. As a specific example, the staining density is quantified with image analysis software "Image J" (Fiji software, open source) so as to be estimated. The evaluation score 1 (WFL status +, weakly positive) stands for the "reciprocal intensity" of 74 to 85 (average 78.5). The evaluation score 2 (WFL status ++, moderately positive) stands for the "reciprocal intensity" of 86 to 104 (average 98.5). The evaluation score 3 (WFL status +++, strongly positive) stands for the "reciprocal intensity" of 105 to 170 (average 132). An increase in the number of samples or populations of measurement data enables a threshold with high reliability. The threshold of the above example has the reciprocal intensity of 86, and the evaluation scores 2 and 3 are grade groups with high recurrence risk.

In addition to the evaluation score given for a quantity of β-GalNAc-biological material in the specimen, the method for acquiring medical care auxiliary information according to an embodiment of the present invention may involve estimating the risk of prostate cancer recurrence by combining at least one piece of information selected from the group consisting of age, Gleason score (GS) or grade group (GG), pathological staging (pT), resection margin (RM), and perineural invasion (pn) obtained from the specimen or a patient from whom the specimen is taken. In the predetermined information, Gleason score (GS) or grade group (GG) and resection margin (RM) are preferable as information for combining the evaluation scores given for a quantity of β-GalNAc-biological material in the specimen.

GS is a value obtained by adding a pattern offered, based on the Gleason grading system, to a tissue image that occupies the largest area of a certain pathological tissue observed with a microscope (primary pattern) and a pattern of a tissue image that occupies the next largest area (secondary pattern, which may be the same as the primary pattern). The Gleason grading system is a five-stage grading system including Pattern 1 to Pattern 5, depending on the morphology and the invasive growth of cancer tissue of prostate cancer. Generally, when GS is 2 to 6, the degree of malignancy is low, when GS is about 7, the degree is medium, and when GS is 8 to 10, the degree is high. Whether GS is 7 or more is a major indication of the degree of malignancy. Furthermore, since 2015, grade group (GG) is used as an indicator of the degree of malignancy in place of GS. GS6 or less corresponds to GG1, GS3 + 4 corresponds to GG2, GS4 + 3 corresponds to GG3, GS8 corresponds to GG4, and GS9 or 10 corresponds to GG5.

Generally, when GS (or GG) is high, that is, when prostate cancer has a high degree of malignancy, the risk of prostate cancer recurrence tends to be high. Accordingly, a predetermined threshold is set for GS (or GG), and for example, when GS is 8 or more (GG is 4 or more), the risk of prostate cancer recurrence is determined to be high.

The pathological staging (pT) is one of the staging methods of TNM (T: primary tumor, N: regional lymph node, M: distant metastasis) and is widely used to indicate the progress level (stage) of prostate cancer. The staging includes clinical staging (pretreatment clinical staging: c) and pathological staging (postoperative histopathological staging: p). The former staging is performed based on information obtained before treatment, and the latter staging is performed when the information is supplemented and corrected based on findings obtained by surgery and histopathological search. In regard to the pathological staging (pT), a primary tumor localized in an organ is represented by pT2, a primary tumor that progresses outside the prostate is represented by pT3, and a primary tumor that invades the bladder and rectum is represented by pT4 (pT1 is not available). Generally, when pT is high, the risk of prostate cancer recurrence tends to be high.

The symbol RM indicates whether resected prostate tissue includes cancer tissue at the proximal margin and the distal margin. Resected prostate tissue with cancer tissue is expressed as the positive tissue (+), and resected prostate tissue without cancer tissue is expressed as negative tissue (-). Generally, when RM is positive, the risk of prostate cancer recurrence tends to be high.

The symbol pn indicates whether resected prostate tissue includes perineural invasion of cancer tissue. Resected prostate tissue with invading cancer tissue is expressed as positive tissue (+), and resected prostate tissue without invading cancer tissue is expressed as negative tissue (-). When pn is positive, the risk of prostate cancer recurrence tends to be high.

There is no particular limitation on how to combine the predetermined information with the medical care auxiliary information based on a quantity (evaluation score) of β-GalNAc-biological material in a tissue specimen according to an embodiment of the present invention. For example, it is preferable to combine by creating nomograms based on the chi-square test. Nomograms for diagnosing prostate cancer based on various kinds of information are known. The present invention may also prepare a nomogram for estimating a risk of prostate cancer recurrence by a method similar to the known methods and may employ the nomogram. Such a nomogram is prepared, taking into consideration a period until the recurrence of prostate cancer.

The risk of prostate cancer recurrence determined by the nomogram utilizing the predetermined information, in addition to the evaluation score of the β-GalNAc-biological material, preferably has higher reliability than the risk of prostate cancer recurrence determined only with the evaluation score of the β-GalNAc-biological material (such as the ROC curve).

### -Kit-

For efficient implementation of the method for acquiring medical care auxiliary information according to an embodiment of the present invention, required reagents may be packed together in a kit. Such a kit includes at least reagents and instruments necessary for measuring a quantity of β-N-acetylgalactosamine-biological material in a prostate tissue specimen, which is carried out in the method for acquiring medical care auxiliary information according to an embodiment of the present invention. In a representative embodiment of the method for measuring a quantity of β-GalNAc-biological material, as described above, the ABC method is used together with the molecule having an affinity for a β-GalNAc residue. Accordingly, a kit suitable for such an embodiment includes, as a main component, a molecule having an affinity for a β-GalNAc residue (lectin having an affinity for a β-GalNAc residue or anti-β-GalNAc antibody), a complex of avidin and biotin-labeled enzyme, and a substrate corresponding to the enzyme (such as DAB). Furthermore, a kit suitable for an embodiment (preferably, the PID method) including a fluorescent nanoparticle instead of a substrate (color-producing agent) such as DAB includes a molecule having an affinity for a β-GalNAc residue and a fluorescent nanoparticle (preferably, PID) modified with avidin or the like.

A kit for carrying out the method for acquiring medical care auxiliary information according to an embodiment of the present invention may include, for example, reagents other than the aforementioned reagents, instruments, and instruction manuals, as needed. Examples of such reagents and instruments include those used for preparing specimen slides of prostate tissue and those used for staining pretreatment, for example, deparaffinization, activation, and blocking. Furthermore, the instruction manual may include information necessary for carrying out the method for acquiring medical care auxiliary information according to an embodiment of the present invention. For example, the instruction manual includes definition of a method (protocol) for using the reagents and instruments, a threshold of the evaluation score of the β-GalNAc-biomolecule, and, if necessary, a threshold used for evaluation of Gleason score (GS) and/or resection margin (RM).

### Examples

With 260 patients diagnosed with prostate cancer and subjected to radical prostatectomy, a concentration of LacdiNAc-PSA in a preoperative serum specimen was quantified. Table 1 shows the backgrounds of the patients.

**[Table 1]**

| Characteristics of PCa patients who underwent RP categorized by WFA-reactivity | | | | | | | | |
|---|---|---|---|---|---|---|---|---|
| **Characteristics** | | **WFA-Reactivity** | | | | | | ***p*** |
| | | **Weakly Positive ^{a}** | | **Moderately** | | **Strongly Positive ^{c}** | | **^{a}vs. ^{b +}** |
| ***n*, total = 260** | | **51** | | **95** | | **112** | | |
| Age, median (range) | | 68 (48-75) | | 68 (56-76) | | 68 (52-78) | | 0.555 |
| PSA ¹, ng/mL, median (range) | | 7.5 (2.3-18.4) | | 7.4 (0.6-27.6) | | 7.5 (0.5-35.9) | | 0.473 |
| Pathological T stage, *n* (%) | | | | | | | | 0.008 ² |
| pT2, *n* = 163 | | 41 | -26.4 | 48 | -29.4 | 72 | -44.2 | 0.002 |
| pT3, *n* = 96 | | 10 | -10.4 | 47 | -49 | 39 | -40.6 | 0.002 |
| pT4, *n* = 1 | | 0 | 0 | 0 | 0 | 1 | -100 | 0.612 |
| Ope GS ³, *n* (%) | Ope GG ⁴ | | | | | | | 0.045 ² |
| 3 + 3, *n* = 11 | Ope GG 1 | 5 | -45.4 | 3 | -27.3 | 3 | -27.3 | 0.035 |
| 3 + 4, *n* = | Ope GG 2 | 28 | -26.5 | 34 | -27.9 | 50 | -44.6 | 0.108 |
| 4 + 3, *n* = 63 | Ope GG 3 | 13 | -19.3 | 28 | -45.2 | 22 | -35.5 | 0.955 |
| 4 + 4, *n* = 9 | Ope GG 4 | 2 | -22.3 | 3 | -33.3 | 4 | -44.4 | 0.889 |
| 3 + 5, *n* = 9 | Ope GG 4 | 1 | -11.1 | 3 | -33.3 | 5 | -55.6 | 0.482 |
| 4 + 5, *n* = 42 | Ope GG 5 | 4 | -9.5 | 17 | -40.5 | 21 | -50 | 0.056 |
| 5 + 4, *n* = 14 | Ope GG 5 | 0 | 0 | 7 | -50 | 7 | -50 | 0.052 |

| pn ⁵, *n* (%) | | | | | | | | |
|---|---|---|---|---|---|---|---|---|
| pn-, *n* = 56 | | 21 | -37.5 | 18 | -32.1 | 17 | -30.4 | <0.001 |
| pn+, *n* = 204 | | 32 | -15.7 | 77 | -37.7 | 95 | -46.6 | <0.001 |
| RM ⁶, *n* (%) | | | | | | | | |
| RM-, *n* = 188 | | 43 | -22.9 | 65 | -34.6 | 80 | -42.5 | 0.108 |
| RM+, *n* = 72 | | 10 | -13.9 | 30 | -41.7 | 32 | -44.4 | 0.108 |

| PSA failure, *n* (%) | | | | | | | | |
|---|---|---|---|---|---|---|---|---|
| -, *n* = 194 | | 49 | -25.3 | 66 | -34 | 79 | -40.7 | <0.001 |
| +, *n* = 66 | | 4 | -6.1 | 29 | -43.9 | 33 | -50 | <0.001 |

| | | | | | | | | |
|---|---|---|---|---|---|---|---|---|
| ¹total PSA; ²X² test; ³Ope GS, Gleason score after radical prostatectomy; ⁴Ope GG, grade group after radical prostatectomy; ⁵pn, perineural invasion; ⁶RM, resection margin; ^{a}weakly positive; ^{b}moderately positive; ^{c}strongly positive. | | | | | | | | |

In Table 1, "pT" represents pathological staging of a primary tumor used as an indicator for the progress level (stage) of prostate cancer. A primary tumor localized in an organ is represented by "pT2," a primary tumor that progresses outside the prostate is represented by "pT3," and a primary tumor that invades the bladder and rectum is represented by "pT4." The symbol "pn" is expressed as "pn+" when perineural invasion is found and is expressed as "pn-" when the perineural invasion is not found.

Staining was carried out according to the ABC method with Wisteria floribunda lectin (WFL). A specific procedure of the staining is described below.

According to a common procedure, a formalin-fixed paraffin-embedded tissue block was prepared from prostate tissue completely removed by surgery. The tissue block was sliced with a microtome to prepare a specimen slide. After deparaffinization according to a common procedure, activation with "Histofine" (Nichirei Bioscience Inc., pH 6.0) and blocking with 0.1% BSA-containing PBS were carried out.

Next, a 100-fold diluted solution of biotinylated WFL (Vector Laboratories) was dripped onto the specimen slide and was allowed to react overnight at 4°C. After the reaction, the "Vectastain ABC kit" (Funakoshi Co., Ltd.) was used, and a complex of avidin and biotin-labeled enzyme and an enzyme substrate solution included in the kit were dropped onto the specimen slide. Then, the mixture was reacted at room temperature for 2 hours.

The stained specimen slide was observed with a bright-field microscope, and a stained image was captured. Using image analysis software "Image J" (Fiji software, open source), the "reciprocal intensity" of each stained slide was measured. Herein, the evaluation score 1 (WFL status +, weakly positive) stands for the "reciprocal intensity" of 74 to 85 (average 78.5). The evaluation score 2 (WFL status ++, moderately positive) stands for the "reciprocal intensity" of 86 to 104 (average 98.5). The evaluation score 3 (WFL status +++, strongly positive) stands for the "reciprocal intensity" of 105 to 170 (average 132). In addition, the stages of the evaluation score were visually observed and affirmed to have a clear difference in staining density. Figs. 2, 3, and 4 illustrate the DAB stained images corresponding to each stage of the evaluation score and the stained images of HE staining (hematoxylin/eosin staining) prepared with specimen slides of adjacent tissue sections for comparison.

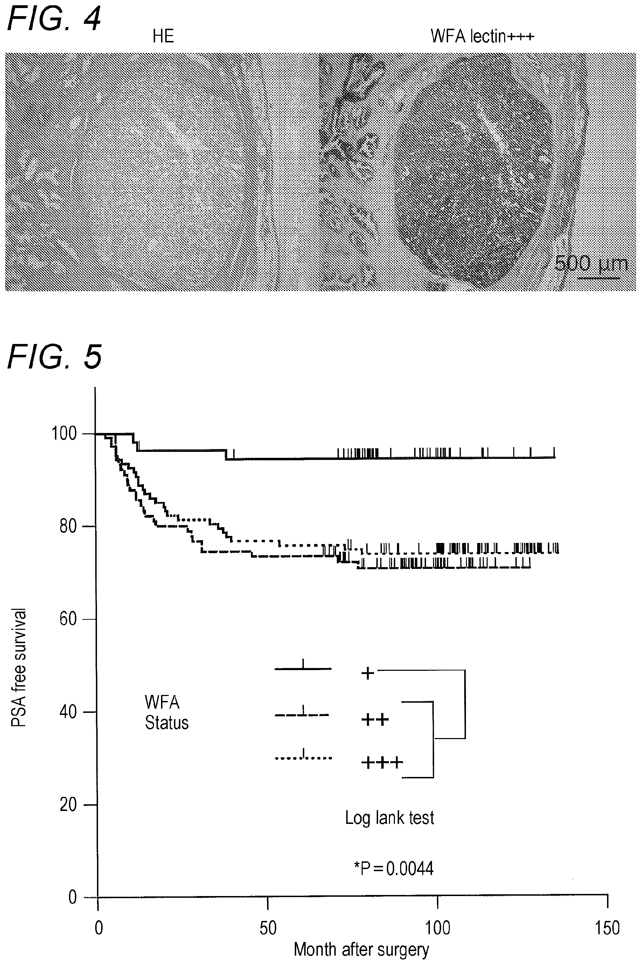

A relationship between the WFL status and the nonrecurrence rate of postoperative prostate cancer was studied according to the Kapran-Meier method, and a significant difference was tested by the log rank test. Fig. 5 shows the results. When the WFL status is +, the nonrecurrence rate is found to be significantly higher than the other groups. In other words, the risk of prostate cancer recurrence is significantly lower than that of the other groups. Conversely, when the WFL status is ++ or +++, the nonrecurrence rate is found to be significantly lower than that when the WFL status is +. In other words, the risk of prostate cancer recurrence is significantly higher.

By multivariate analysis (logistic regression analysis), the present inventors have studied whether each histopathological parameter shown in Table 1 is a risk factor for prostate cancer recurrence. Table 2 shows the results. The WFL status +++ proves to be an independent risk factor for prostate cancer recurrence. Furthermore, the status with GS8 or more and RM+ are also found to be risk factors for prostate cancer recurrence. Therefore, it becomes clear that, in addition to the WFL status, GS and RM are also used for estimating a risk of prostate cancer recurrence.

**[Table 2]**

| Multivariate analysis to determine independent predictor of PSA recurrence | | | | |
|---|---|---|---|---|
| variable | odds ratio | std. error | z-score | *P* value |
| Age | 1.076 | 0.034 | 2.312 | 0.990 |
| WFA status ++ | 3.033 | 1.949 | 1.727 | 0.042 |
| WFA status +++ | 3.092 | 1.941 | 1.798 | 0.036 |
| pT ≥ 3 | 1.733 | 0.686 | 1.389 | 0.082 |
| Ope GS ≥ 8 | 2.114 | 0.700 | 2.262 | 0.024 |
| RM+ | 3.008 | 1.221 | 2.271 | 0.007 |
| pn+ | 2.498 | 1.341 | 1.705 | 0.088 |

| | | | | |
|---|---|---|---|---|
| pT: pathological stage, Ope GS: Gleason score after RP, RM: resection margin, pn: perineural invasion | | | | |

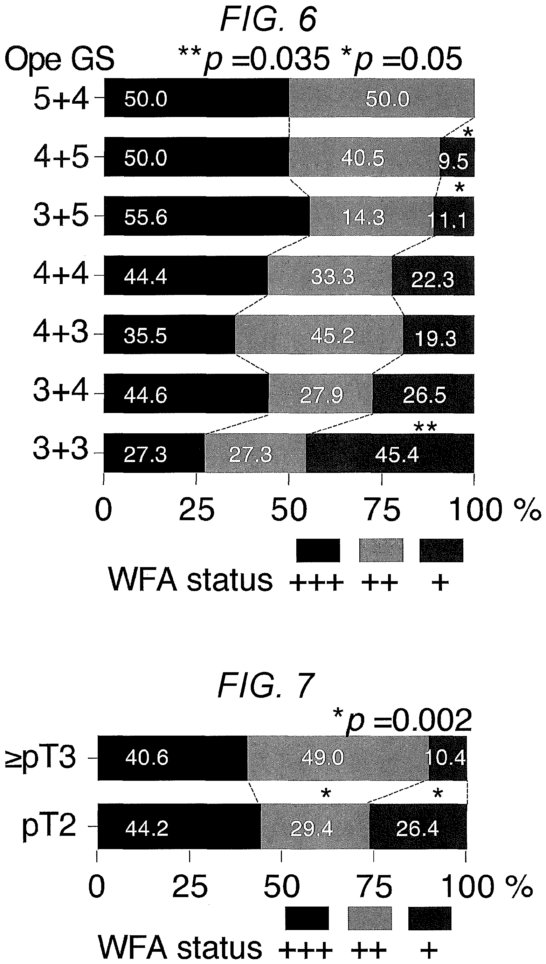

A relationship between the WFL status and each of GS, pT, and pn was studied. Figs. 6, 7, and 8 show the results. In the group with high GS, the group with pT3 or more, and the group with pn+, it is found that the percentage of the WFL status + is lower and the percentage of WFL status ++ or +++ is higher than the other groups.

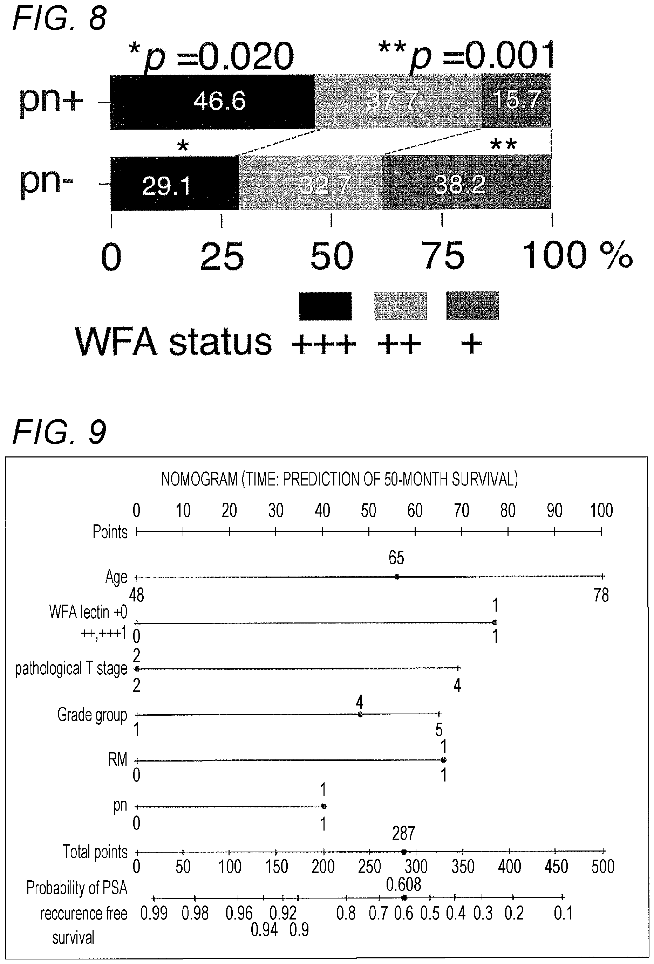

With the WFL status, the age of patients, the grade group (GG, converted from GS), pT, RM, and pn, the Cox proportional hazards regression analysis was carried out, and a nomogram was prepared based on the chisquared test (Wald test). The data used to create the nomogram is shown in Table 3. Furthermore, Fig. 9 illustrates the prepared nomogram and, as an example, the prediction of 50-month survival when certain patient's data is applied to the nomogram. A method for using the nomogram is described below. (i) Plot the predetermined data (factor) in each bar. (ii) Draw a perpendicular from each plot point to the bar of "points," and regard the intersection as the point of each factor. (iii) Plot the total value of each point on the bar of "Total points". (iv) Draw a perpendicular from the plot point of "Total points" to the bar of "Probability of PSA recurrence free survival," and regard the intersection as the survival probability 50 months after prostate cancer recurrence.

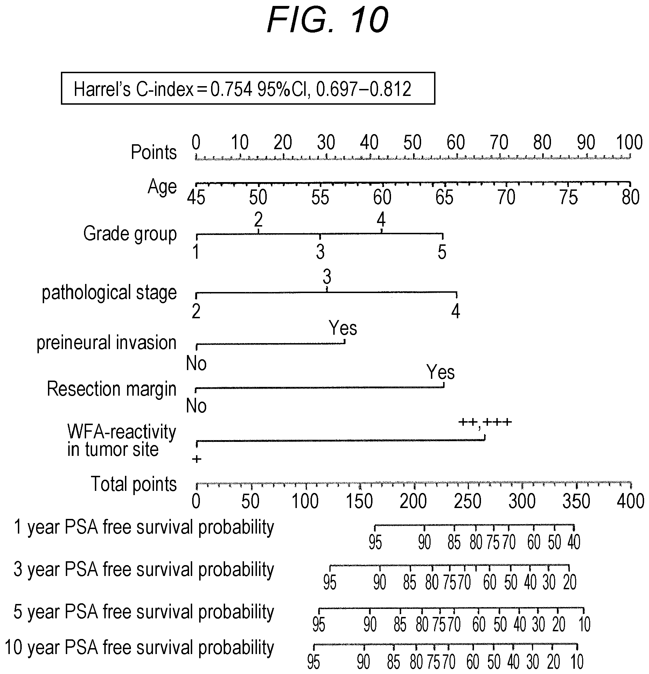

Furthermore, Fig. 10 illustrates the predictions of 1-, 3-, 5-, and 10-year survival which replace the prediction of 50-month survival illustrated in Fig. 9.

**[Table 3]**

| Covariance | Coefficient | Standard error | Wald test Chi-square value | Degree of freedom | P value | *: |
|---|---|---|---|---|---|---|
| | | | | | | P<0.05 |
| | | | | | | **: |
| | | | | | | P<0.01 |
| Age | 0.0448 | 0.0272 | 2.7254 | 1 | 0.0988 | |
| WFA lectin +0 | | | | | | |
| ++,+++1 | 1.0403 | 0.5287 | 3.8719 | 1 | 0.0491 | * |
| pT | 0.4636 | 0.3364 | 1.8989 | 1 | 0.1682 | |
| Grade group | 0.2199 | 0.0993 | 4.9080 | 1 | 0.0267 | * |
| RM | 0.8851 | 0.3192 | 7.6911 | 1 | 0.0055 | ** |

## Claims

1. A method for acquiring medical care auxiliary information for estimating a risk of prostate cancer recurrence with a specimen of prostate tissue,
wherein an at least three-stage evaluation score is used to express a quantity of biological material in a tumor site of the specimen, the biological material having a β-N-acetylgalactosamine residue at a nonreducing terminal of a sugar chain.

2. The method for acquiring medical care auxiliary information according to claim 1, wherein, when a quantity of biological material is equal to or more than a threshold, the biological material is classified into a grade group with high recurrence risk of the evaluation score, and when a quantity of biological material is less than the threshold, the biological material is classified into a grade group with low recurrence risk of the evaluation score.

3. The method for acquiring medical care auxiliary information according to claim 1 or 2, wherein the evaluation score is obtained in regard to a stained specimen which is prepared by a staining method that involves binding a molecule having an affinity for a β-N-acetylgalactosamine residue to the biological material.

4. The method for acquiring medical care auxiliary information according to claim 3, wherein the molecule having an affinity for a β-N-acetylgalactosamine residue is Wisteria floribunda lectin (WFA), Soybean Agglutinin (SBA) or Vicia Villosa Lectin (VVL), or an anti-β-N-acetylgalactosamine antibody.

5. The method for acquiring medical care auxiliary information according to claim 3 or 4, wherein the staining method is performed by the Avidin Biotinylated Enzyme Complex (ABC) method.

6. The method for acquiring medical care auxiliary information according to any one of claims 1 to 5, the method comprising
estimating a risk of prostate cancer recurrence by combining at least one piece of information selected from a group consisting of age, Gleason score (GS) or grade group (GG), pathological staging (pT), resection margin (RM), and perineural invasion (pn) obtained from the specimen or a patient from whom the specimen is taken.
